# EUROPEAN PATENT SPECIFICATION

(11) **EP 3 323 868 B1**
(45) Date of publication and mention of the grant of the patent: **13.05.2020**
(21) Application number: 16824545.4
(22) Date of filing: 15.07.2016
(51) Int. Cl.: C09K 3/00, A61K 8/44, A61K 47/16, A61Q 19/00, C07C 235/74

(54) **GEL COMPOSITION CONTAINING ACYL BASIC AMINO ACID DERIVATIVE**
GELZUSAMMENSETZUNG MIT EINEM BASISCHEN ACYLAMINOSÄUREDERIVAT
COMPOSITION DE GEL CONTENANT UN DÉRIVÉ D'ACIDE ACYL AMINÉ BASIQUE

(30) Priority: 16.07.2015 JP 2015142305
(43) Date of publication of application: 23.05.2018
(73) Proprietor: AJINOMOTO CO., INC., Chuo-ku Tokyo 104-8315 (JP)
(72) Inventor: KOBAYASHI, Shun, Kawasaki-shi Kanagawa 210-8681 (JP)
(74) Representative: Mewburn Ellis LLP
(86) International application number: PCT/JP2016/071001
(87) International publication number: WO 2017/010565

(56) References cited:
- EP-A1- 1 473 027
- EP-A1- 2 813 487
- WO-A1-2013/118896
- JP-A- 2004 323 505
- SUZUKI, MASAHIRO ET AL.: 'New gemini organogelators linked by oxalyl amide: organogel formation and their thermal stabilities' TETRAHEDRON LETTERS vol. 44, no. 36, 2003, ISSN 0040-4039 pages 6841 - 6843, XP004444415
- SUZUKI, MASAHIRO ET AL.: 'L-Lysine based gemini organogelators: their organogelation properties and thermally stable organogels' ORGANIC & BIOMOLECULAR CHEMISTRY vol. 1, no. 22, 2003, ISSN 1477-0520 pages 4124 - 4131, XP002276454
- SUZUKI, MASAHIRO ET AL.: 'Novel dumbbell-form low-molecular-weight gelators based on L-lysine: their hydrogelation and organogelation properties' NEW JOURNAL OF CHEMISTRY vol. 29, no. 11, 09 September 2005, ISSN 1144-0546 pages 1439 - 1444, XP055346030

## Description

### [Technical Field]

The present invention relates to a gel composition having high stability in a wide pH region.

### [Background Art]

A method for controlling flowability of a composition which is liquid at ambient temperature, such as cosmetics, pharmaceutical products, agricultural chemicals, feeds, fertilizers, paints etc., and processing same into a form that meets diversified use objects is an industrially highly important technique. When the flowability of an aqueous composition is controlled, water-soluble polymers such as carboxyvinyl polymer, xanthan gum and the like are generally used. In a system in which salt is present, gelling is difficult, and a large amount of a water-soluble polymer needs to be added.

In patent documents 1 and 2, a gelling agent capable of gelling by the addition of a small amount thereof was studied, and patent document 1 disclose a basic amino acid derivative represented by the below-mentioned formula (1) as a gelling agent, and patent document 2 discloses a basic amino acid derivative represented by the below-mentioned formula (2B) or a salt thereof.

### [Document List]

### [Patent documents]

patent document 1: WO 2004/096754
patent document 2: WO 2013/118896

### [SUMMARY OF THE INVENTION]

### [Problems to be Solved by the Invention]

The present inventor has found that the basic amino acid derivative disclosed in patent document 1 and the basic amino acid derivative disclosed in patent document 2 can merely gelate an aqueous composition in a particularly narrow pH region and cannot gelate same in a wide pH region; and since they produce a cloudy gel, there is a room for improvement for use in a transparent formulation.

Accordingly, the problem of the present invention is to provide a gel composition that can exist stably in a gel state in a wide pH range, in which white turbidity is suppressed.

### [Means of Solving the Problems]

The present inventor has conducted intensive studies in an attempt to solve the aforementioned problems and found that an aqueous composition can be gelated stably
in a wide pH region and the white turbidity of the gel can be suppressed by using, as a gelling agent, a compound represented by the following formula (1) or a salt thereof, and a compound represented by the following formula (2B) or a salt thereof in combination, which resulted in the completion of the present invention.

That is, the present invention provides the following.
[1] A gel composition comprising
   (A) a compound represented by the formula (1): 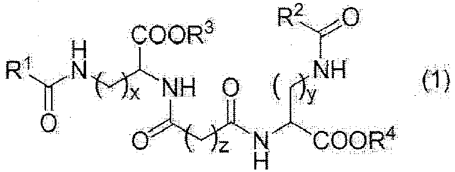 wherein
      R¹ and R² are each independently an alkyl group having 5 - 21 carbon atoms or an alkenyl group having 5 - 21 carbon atoms,
      R³ and R⁴ are each independently a hydrogen atom, an alkyl group having 1 - 22 carbon atoms or an alkenyl group having 2 - 22 carbon atoms,
      z is an integer of not less than 0, and
      x and y are each independently an integer of 2 - 4, or a salt thereof;
   (B) a compound represented by the formula (2B): 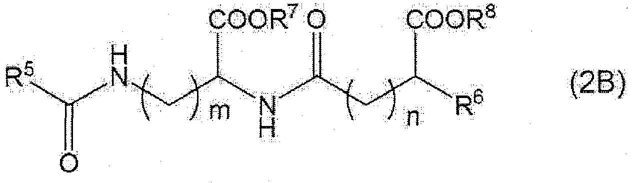 wherein
      R⁵ is an alkyl group having 5 - 21 carbon atoms or an alkenyl group having 5 - 21 carbon atoms,
      R⁶ is a hydrogen atom or an optionally substituted hydrocarbon group,
      m is an integer of 1 - 4,
      n is an integer of 0 - 15, and
      R⁷ and R⁸ are each independently a hydrogen atom or an optionally substituted, saturated or unsaturated, straight chain or branched chain hydrocarbon group having 1 - 6 carbon atoms, or a salt thereof, and
   (C) water.
[2] The composition of the above-mentioned [1], wherein z is an integer of 0 - 10.
[3] The composition of the above-mentioned [1] or [2], wherein z is an integer of 7 or 8.
[4] The composition of any of the above-mentioned [1] - [3], wherein x and y are each 4.
[5] The composition of any of the above-mentioned [1] - [4], wherein R¹ and R² are each independently a straight chain alkyl group having 5 - 15 carbon atoms.
[6] The composition of any of the above-mentioned [1] - [5], wherein R³ and R⁴ are each a hydrogen atom.
[7] The composition of any of the above-mentioned [1] - [6], wherein R⁵ is a straight chain alkyl group having 5 - 15 carbon atoms.
[8] The composition of any of the above-mentioned [1] - [7], wherein R⁶ is a hydrogen atom or an optionally substituted, saturated or unsaturated, straight chain or branched chain hydrocarbon group having 1 - 30 carbon atoms.
[9] The composition of any of the above-mentioned [1] - [8], wherein n is an integer of 0 - 9.
[10] The composition of any of the above-mentioned [1] - [9], wherein R⁷ and R⁸ are each independently a hydrogen atom or a saturated or unsaturated, straight chain or branched chain hydrocarbon group having 1 - 6 carbon atoms.
[11] The composition of any of the above-mentioned [1] - [10], wherein m is 4.
[12] The composition of any of the above-mentioned [1] - [11], wherein component (A) is bis(Nε-lauroyl-L-lysine)sebacoylamide, and
   component (B) is Nε-lauroyl-Nα-(9-carboxynonanoyl)-L-lysine.
[13] The composition of any of the above-mentioned [1] - [12], having a pH of 5 - 7.
[14] The composition of any of the above-mentioned [1] - [13], wherein a weight ratio of (A) a compound represented by the formula (1) or a salt thereof and (B) a compound represented by the formula (2B) or a salt thereof ((A):(B)) is 99:1 - 50:50.
[15] The composition of any of the above-mentioned [1] - [14], which is a cosmetic.
[16] The composition of any of the above-mentioned [1] - [15], wherein R⁵ is a straight chain alkyl group having 5 - 15 carbon atoms, and
   R⁶ is a hydrogen atom or an optionally substituted, saturated or unsaturated, straight chain or branched chain hydrocarbon group having 1 - 30 carbon atoms.
[17] The composition of any of the above-mentioned [1] - [16], wherein R⁶ is a hydrogen atom or a saturated or unsaturated, straight chain or branched chain hydrocarbon group having 1 - 15 carbon atoms,
   n is an integer of 0 - 9, and
   R⁷ and R⁸ are each independently a hydrogen atom or a saturated or unsaturated, straight chain or branched chain hydrocarbon group having 1 - 6 carbon atoms.
[18] The composition of any of the above-mentioned [1] - [17], wherein R⁵ is an undecyl group, and m is 4.
[19] A gelling agent comprising
   (A) a compound represented by the formula (1): 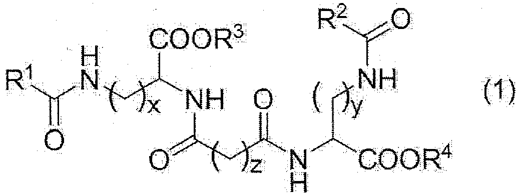 wherein
      R¹ and R² are each independently an alkyl group having 5 - 21 carbon atoms or an alkenyl group having 5 - 21 carbon atoms,
      R³ and R⁴ are each independently a hydrogen atom, an alkyl group having 1 - 22 carbon atoms or an alkenyl group having 2 - 22 carbon atoms,
      z is an integer of not less than 0, and
      x and y are each independently an integer of 2 - 4, or a salt thereof; and
   (B) a compound represented by the formula (2B): 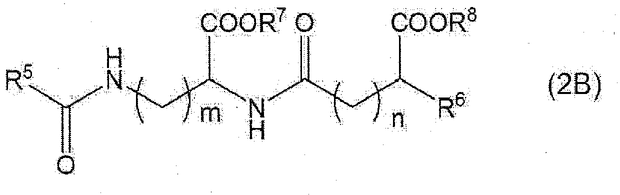 wherein
      R⁵ is an alkyl group having 5 - 21 carbon atoms or an alkenyl group having 5 - 21 carbon atoms,
      R⁶ is a hydrogen atom or an optionally substituted hydrocarbon group,
      m is an integer of 1 - 4,
      n is an integer of 0 - 15, and
      R⁷ and R⁸ are each independently a hydrogen atom or an optionally substituted, saturated or unsaturated, straight chain or branched chain hydrocarbon group having 1 - 6 carbon atoms, or a salt thereof.
[20] The gelling agent of the above-mentioned [19], wherein z is an integer of 0 - 10.
[21] The gelling agent of the above-mentioned [19] or [20], wherein z is an integer of 7 or 8.
[22] The gelling agent of any of the above-mentioned [19]-[21], wherein x and y are each 4.
[23] The gelling agent of any of the above-mentioned [19]-[22], wherein R¹ and R² are each independently a straight chain alkyl group having 5 - 15 carbon atoms.
[24] The gelling agent of any of the above-mentioned [19]-[23], wherein R³ and R⁴ are each a hydrogen atom.
[25] The gelling agent of any of the above-mentioned [19]-[24], wherein R⁵ is a straight chain alkyl group having 5 - 15 carbon atoms.
[26] The gelling agent of any of the above-mentioned [19]-[25], wherein R⁶ is a hydrogen atom or an optionally substituted, saturated or unsaturated, straight chain or branched chain hydrocarbon group having 1 - 30 carbon atoms.
[27] The gelling agent of any of the above-mentioned [19]-[26], wherein n is an integer of 0 - 9.
[28] The gelling agent of any of the above-mentioned [19]-[27], wherein R⁷ and R⁸ are each independently a hydrogen atom or a saturated or unsaturated, straight chain or branched chain hydrocarbon group having 1 - 6 carbon atoms.
[29] The gelling agent of any of the above-mentioned [19]-[28], wherein m is 4.
[30] The gelling agent of any of the above-mentioned [19]-[29], wherein component (A) is bis(Nε-lauroyl-L-lysine)sebacoylamide, and
   component (B) is Nε-lauroyl-Nα-(9-carboxynonanoyl)-L-lysine.
[31] The gelling agent of any of the above-mentioned [19]-[30], wherein a weight ratio of (A) a compound represented by the formula (1) or a salt thereof and (B) a compound represented by the formula (2B) or a salt thereof ((A):(B)) is 99:1 - 50:50.
[32] The gelling agent of any of the above-mentioned [19]-[31], wherein R⁵ is a straight chain alkyl group having 5 - 15 carbon atoms, and
   R⁶ is a hydrogen atom or an optionally substituted, saturated or unsaturated, straight chain or branched chain hydrocarbon group having 1 - 30 carbon atoms.
[33] The gelling agent of any of the above-mentioned [19]-[32], wherein R⁶ is a hydrogen atom or a saturated or unsaturated, straight chain or branched chain hydrocarbon group having 1 - 15 carbon atoms,
   n is an integer of 0 - 9, and
   R⁷ and R⁸ are each independently a hydrogen atom or a saturated or unsaturated, straight chain or branched chain hydrocarbon group having 1 - 6 carbon atoms.
[34] The gelling agent of any of the above-mentioned [19]-[33], wherein R⁵ is an undecyl group, and m is 4.

### [Effect of the Invention]

According to the present invention, a gel composition having high stability in a wide pH region can be provided. According to the present invention, moreover, a gel composition in which white turbidity of the gel is suppressed can be provided.

### [Description of Embodiments]

The present invention is explained in detail in the following.

The gel composition of the present invention contains (A) a compound represented by the above-mentioned formula (1) or a salt thereof (hereinafter to be also referred to as component (A)), (B) a compound represented by the above-mentioned formula (2B) or a salt thereof (hereinafter to be also referred to as component (B)), and (C) water (hereinafter to be also referred to as component (C)).

Component (A) is explained in the following.

R¹ and R² are each independently an alkyl group having 5 - 21 carbon atoms or an alkenyl group having 5 - 21 carbon atoms.

An alkyl group having 5 - 21 carbon atoms means a straight chain or branched alkyl group having 5 - 21 carbon atoms. Specific examples thereof include pentyl group, isopentyl group, neopentyl group, hexyl group, isohexyl group, neohexyl group, heptyl group, isoheptyl group, neoheptyl group, octyl group, isooctyl group, nonyl group, isononyl group, decyl group, isodecyl group, undecyl group, dodecyl group, tridecyl group, tetradecyl group, pentadecyl group, hexadecyl group, heptadecyl group, octadecyl group, nonadecyl group, icosyl group and the like.

An alkenyl group having 5 - 21 carbon atoms means a straight chain or branched alkenyl group having 5 - 21 carbon atoms. Specific examples thereof include pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

An alkyl group having 5 - 15 carbon atoms means a straight chain or branched alkyl group having 5 - 15 carbon atoms. Specific examples thereof include pentyl group, hexyl group, heptyl group, octyl group, nonyl group, decyl group, undecyl group, dodecyl group, tridecyl group, tetradecyl group, pentadecyl group and the like.

An alkyl group having 7 - 11 carbon atoms means a straight chain or branched alkyl group having 7 - 11 carbon atoms. Specific examples thereof include heptyl group, octyl group, nonyl group, decyl group, undecyl group and the like.

Preferably, R¹ and R² are each independently an alkyl group having 5 - 15 carbon atoms, more preferably each independently an alkyl group having 7 - 11 carbon atoms.

Preferably, R¹ and R² are each a straight chain alkyl group. Furthermore, R¹ and R² are preferably the same.

R³ and R⁴ are each independently a hydrogen atom, an alkyl group having 1 - 22 carbon atoms or an alkenyl group having 2 - 22 carbon atoms.

An alkyl group having 1 - 22 carbon atoms means a straight chain or branched alkyl group having 1 - 22 carbon atoms. Specific examples thereof include methyl group, ethyl group, propyl group, isopropyl group, butyl group, isobutyl group, sec-butyl group, tert-butyl group, pentyl group, isopentyl group, neopentyl group, hexyl group, isohexyl group, neohexyl group, heptyl group, isoheptyl group, neoheptyl group, octyl group, isooctyl group, nonyl group, isononyl group, decyl group, isodecyl group, undecyl group, dodecyl group, tridecyl group, tetradecyl group, pentadecyl group, hexadecyl group, heptadecyl group, octadecyl group, nonadecyl group, icosyl group and the like.

An alkenyl group having 2 - 22 carbon atoms means a straight chain or branched alkenyl group having 2 - 22 carbon atoms. Specific examples thereof include ethenyl group, 1-propenyl group, 2-propenyl group, 1-butenyl group, 2-butenyl group, 3-butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

Preferably, both R³ and R⁴ are hydrogen atoms.

z is an integer of not less than 0, more preferably 0 - 10, particularly preferably 7 and 8.

x and y are each independently an integer of 2 - 4, preferably both 4.

As a compound represented by the formula (1), preferably, the following compounds can be mentioned.

### (Compound A)

A compound wherein R¹ and R² are each independently a straight chain alkyl group having 5 - 15 carbon atoms,
both R³ and R⁴ are hydrogen atoms,
z is an integer of 0 - 10, and
both x and y are 4.

### (Compound B)

A compound wherein both R¹ and R² are straight chain alkyl groups having 5 - 15 carbon atoms,
both R³ and R⁴ are hydrogen atoms,
z is 7 or 8, and
both x and y are 4.

### (Compound C)

A compound wherein both R¹ and R² are straight chain alkyl groups having 7 - 11 carbon atoms,
both R³ and R⁴ are hydrogen atoms,
z is 7 or 8, and
both x and y are 4.

Specific examples of a compound represented by the formula (1) include
bis(Nε-lauroyl-L-lysine)sebacoylamide, and
bis(Nε-octanoyl-L-lysine)sebacoylamide,
or a salt thereof.

A salt of a compound represented by the formula (1) is not particularly limited. Examples thereof include inorganic salts such as alkali metal salts (e.g., as sodium salt, potassium salt and the like), alkaline earth metal salts (e.g., calcium salt, magnesium salt and the like), aluminum salt, salt with zinc and the like, and organic salts such as salts with organic amine such as ammonia, monoethanolamine, diethanolamine, triethanolamine and the like, salts with basic amino acid such as arginine, lysine and the like, and the like. One kind of these may be used, or two or more kinds selected from the above-mentioned group may be used in a mixture. From the aspects of easy availability, handling property and the like, alkali metal salt, organic amine salt, or basic amino acid salt is preferable, and sodium salt and potassium salt are particularly preferable.

The compound represented by the formula (1) (hereinafter to be also referred to as compound (1)) for component (A) in the present invention can be produced by the method described in the aforementioned patent document 1.

Compound (1) can be produced by a conventional method (JP-A-2004-323505, Org. Biomol. Chem., 2003, 1, 4124-4131, New J. Chem., 2005, 29, 1439-1444 etc.). For example, as shown in the following formula, a symmetric type compound (1') among compounds (1) can be produced by reacting Nω-acylamino acid (2) and dicarboxylic acid dichloride (3) in an appropriate solvent. 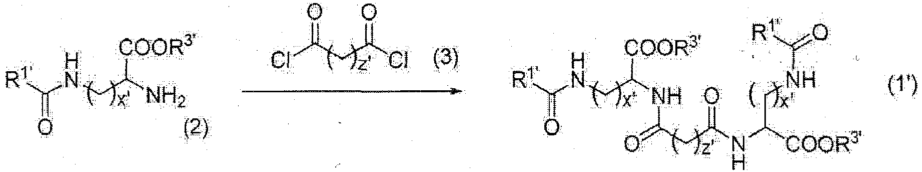 wherein R^{1'} is an alkyl group having 5 - 21 carbon atoms or an alkenyl group having 5 - 21 carbon atoms, R^{3'} is a hydrogen atom, an alkyl group having 1 - 22 carbon atoms or an alkenyl group having 2 - 22 carbon atoms, z' is an integer of not less than 0, and x' is an integer of 2 - 4.

Examples of the Nω-acylamino acid (2) include Nε-acyl lysine (e.g., Nε-hexanoyl-L-lysine, Nε-octanoyl-L-lysine, Nε-lauroyl-L-lysine etc.), Nδ-acyl ornithine (e.g., Nδ-hexanoyl-L-ornithine etc.), Nγ-acyl-α,γ-diaminobutyric acid and the like.

Examples of the dicarboxylic acid dichloride (3) include oxalyl chloride, malonyl chloride, succinyl chloride, glutaryl chloride, adipoyl chloride, pimeloyl chloride, suberoyl chloride, azelaoyl chloride, sebacoyl chloride, dodecanedioyl chloride and the like. The amount of dicarboxylic acid dichloride (3) to be used is generally 0.4 - 0.6 equivalent relative to Nω-acylamino acid (2).

While the solvent is not particularly limited as long as it is inert to the reaction, examples thereof include ethers such as diethyl ether, tetrahydrofuran and the like.

Of compounds (1), an asymmetric type compound (1") can be produced as follows. First, Nω-acylamino acid (2) and dicarboxylic acid monochloride monoester (4) are reacted in an appropriate solvent to give compound (5) (step 1). Then, the primary ester moiety of the obtained compound (5) is hydrolyzed in the presence of a base such as sodium hydroxide, potassium hydroxide and the like, the carboxylic acid moiety is chlorinated with a chlorinating agent such as thionyl chloride and the like, and the compound is reacted with Nω-acylamino acid (2') which is different from Nω-acylamino acid (2) used in the aforementioned step 1, whereby derivative (1") can be produced (step 2). 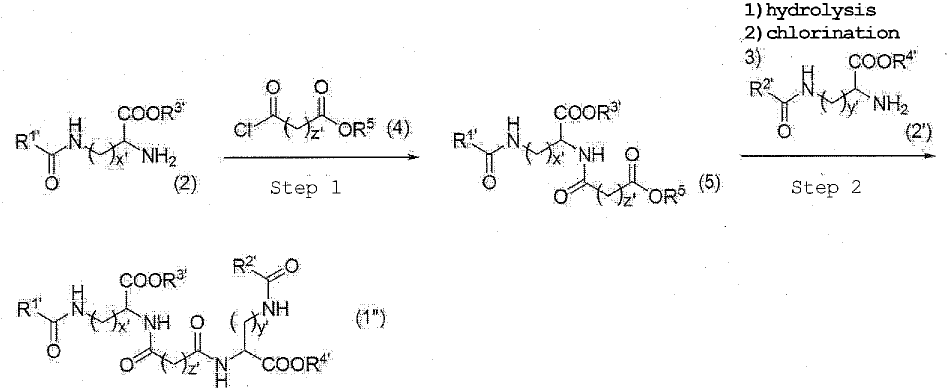 wherein R^{1'}, R^{3'}, z' and x' are as defined above, R^{2'} is an alkyl group having 5 - 21 carbon atoms or an alkenyl group having 5 - 21 carbon atoms, R^{4'} is a hydrogen atom, an alkyl group having 1 - 22 carbon atoms or an alkenyl group having 2 - 22 carbon atoms, R⁵ is an alkyl group such as methyl group, ethyl group and the like, and y' is an integer of 2 - 4.

As Nω-acylamino acids (2) and (2'), Nω-acylamino acids similar to those mentioned above can be used.

As dicarboxylic acid monochloride monoester (4), a commercially available product can be used as is when it is commercially available, or one produced by a method known per se or a method analogous thereto can also be used.

A acyl basic amino acid derivative obtained by the above-mentioned method can be converted to a salt of the acyl basic amino acid derivative by a reaction with alkali metal hydroxide such as sodium hydroxide, potassium hydroxide and the like, alkali earth metal hydroxide such as calcium hydroxide and the like, organic amine base, or the like.

As the Nω-acylamino acid, Nε-acyllysine, Nδ-acylornithine, and Nγ-acyl-α,γ-diaminobutyric acid can be mentioned, from which Nε-acyllysine is most preferable.

Nω-acylamino acid can be easily synthesized by, for example, heating dehydrating of basic amino acid and long chain fatty acid. As industrially available Nω-acylamino acid, AMIHOPE LL (Nε-lauroyl-L-lysine) manufactured by Ajinomoto Co., Inc. and the like can be mentioned.

While Nω-acylamino acid may be any of an optically active form and a racemate, an optically active form is more preferable since gelation ability is improved. These may be used alone, or two or more kinds thereof may be used in a mixture.

Component (B) is explained next.

Examples of the "hydrocarbon group" in the present specification include (i) chain hydrocarbon groups such as alkyl groups such as methyl group, ethyl group, propyl group, isopropyl group, butyl group, isobutyl group, sec-butyl group, tert-butyl group, pentyl group, isopentyl group, sec-pentyl group, tert-pentyl group, hexyl group, heptyl group, octyl group and the like, alkenyl groups such as vinyl group, 1-propen-1-yl group, 2-propen-1-yl group, isopropenyl group, 2-buten-1-yl group, 4-penten-1-yl group, 5-hexen-1-yl group and the like, alkynyl groups such as ethynyl group, 1-propyn-1-yl group, 2-propyn-1-yl group, 4-pentyn-1-yl group, 5-hexyn-1-yl group and the like, and the like; (ii) alicyclic hydrocarbon groups such as cycloalkyl groups such as cyclopropyl group, cyclobutyl group, cyclopentyl group and the like, cycloalkenyl groups such as cyclopropenyl group, cyclobutenyl group, cyclopentenyl group and the like, and the like; and (iii) aromatic hydrocarbon groups such as aryl groups such as phenyl group, naphthyl group and the like, and the like.

Examples of the "halogen atom" in the present specification include fluorine atom, chlorine atom, bromine atom, and iodine atom.

In the present specification, as the substituent of the chain hydrocarbon group among the hydrocarbon groups, a substituent selected from the following substituent group A can be mentioned. In addition, as the substituent of the cyclic hydrocarbon group among the hydrocarbon groups, a substituent selected from the following substituent group A and substituent group B can be mentioned. The number of the substituents is one to the maximum substitutable number, more preferably 1 - 3, further preferably 1.

In the present specification, substituent group A includes
(a) halogen atom;
(b) hydroxy group;
(c) nitro group;
(d) cyano group;
(e) C₃₋₇ cycloalkyl group;
(f) C₁₋₆ alkoxy group;
(g) C₃₋₇ cycloalkyloxy group;
(h) amino group optionally mono- or di-substituted by a substituent selected from the group consisting of C₁₋₆ alkyl group and C₂₋₆ alkenyl group;
(i) C₁₋₆ alkyl-carbonyl group;
(j) C₃₋₇ cycloalkyl-carbonyl group; and
(k) mono- or di-C₁₋₆ alkyl-carbamoyl group.

In the present specification, substituent group B includes
(a) C₁₋₁₀ alkyl group;
(b) C₂₋₁₀ alkenyl group; and
(c) C₂₋₁₀ alkynyl group.

R⁵ is an alkyl group having 5 - 21 carbon atoms or an alkenyl group having 5 - 21 carbon atoms.

As the alkyl group or alkenyl group for R⁵, the alkyl groups and alkenyl groups exemplified as the above-mentioned R¹ and R² can be mentioned.

R⁵ is preferably an alkyl group having 5 - 15 carbon atoms, more preferably an alkyl group having 7 - 11 carbon atoms.

In addition, R⁵ is preferably a straight chain alkyl group.

R⁶ is a hydrogen atom or an optionally substituted hydrocarbon group, preferably, a hydrogen atom or an optionally substituted saturated or unsaturated straight chain or branched chain hydrocarbon group, more preferably, a hydrogen atom or an optionally substituted, saturated or unsaturated, straight chain or branched chain hydrocarbon group having 1 - 30 carbon atoms. Examples of the saturated or unsaturated, straight chain or branched chain hydrocarbon group having 1 - 30 carbon atoms include methyl group, ethyl group, isopropyl group, propyl group, butyl group, isobutyl group, sec-butyl group, tert-butyl group, pentyl group, sec-pentyl group, tert-pentyl group, isopentyl group, hexyl group, heptyl group, octyl group, 2-ethylhexyl group, tert-octyl group, nonyl group, isononyl group, decyl group, isodecyl group, undecyl group, dodecyl group, tridecyl group, isotridecyl group, tetradecyl group, and pentadecyl group and the like.

R⁶ is preferably a hydrogen atom or a saturated or unsaturated, straight chain or branched chain hydrocarbon group having 1 - 15 carbon atoms, more preferably, a hydrogen atom or a saturated or unsaturated, straight chain or branched chain hydrocarbon group having 4 - 15 carbon atoms, further preferably, a hydrogen atom or a saturated or unsaturated, straight chain or branched chain hydrocarbon group having 4 - 10 carbon atoms, particularly preferably, a hydrogen atom or a saturated straight chain or branched chain hydrocarbon group having 7 - 10 carbon atoms.

m is an integer of 1 - 4. When m=3, an ornithine derivative is obtained, and when m=4, a lysine derivative is obtained. From the aspects of gelation ability and easiness of preparing, m is preferably 3 or 4, and 4 (lysine derivative) is particularly preferable.

In the formula (2B), n is an integer of 0 - 15, and an integer of 0 - 9 is preferable.

The total carbon number of R⁶ and (CH₂)n is desirably an appropriate carbon number from the aspects of gelling ability and solubility. The total carbon number of R⁶ and (CH₂)n is preferably 3 - 12, more preferably 3 - 11, further preferably 5 - 11, further more preferable 7 - 11. For example, when n=1, R⁶ is preferably a saturated or unsaturated, straight chain or branched chain hydrocarbon group having 2 - 11 carbon atoms, more preferably hexyl group, heptyl group, octyl group, or decyl group, further preferably heptyl group or decyl group. When R⁶ is a hydrogen atom, n is preferably 3 - 12, more preferably 3 - 7.

R⁷ and R⁸ are each independently a hydrogen atom or an optionally substituted, saturated or unsaturated, straight chain or branched chain hydrocarbon group having 1 - 6 carbon atoms, preferably, each independently a hydrogen atom or a saturated or unsaturated, straight chain or branched chain hydrocarbon group having 1 - 6 carbon atoms.

Examples of the "saturated or unsaturated, straight chain or branched chain hydrocarbon group having 1 - 6 carbon atoms" include methyl group, ethyl group, isopropyl group, propyl group, butyl group, isobutyl group, sec-butyl group, tert-butyl group, pentyl group, sec-pentyl group, tert-pentyl group, isopentyl group, hexyl group and the like. From the aspect of solubility, R⁷ and R⁸ are preferably each independently a hydrogen atom or a saturated or unsaturated, straight chain or branched chain hydrocarbon group having 1 - 4 carbon atoms, R⁷ is more preferably a hydrogen atom, a methyl group or an ethyl group, and R⁸ is further preferably a hydrogen atom.

As a compound represented by the formula (2B), the following compounds can be preferably mentioned.

### (compound-A)

A compound wherein R⁵ is a straight chain alkyl group having 5 - 15 carbon atoms,
R⁶ is a hydrogen atom or a saturated or unsaturated, straight chain or branched chain hydrocarbon group having 4 - 10 carbon atoms,
m is an integer of 1 - 4,
n is an integer of 0 - 9, and
R⁷ and R⁸ are each independently a hydrogen atom or a saturated or unsaturated, straight chain or branched chain hydrocarbon group having 1 - 4 carbon atoms.

### (compound-B)

A compound wherein R⁵ is an undecyl group, a tridecyl group, a pentadecyl group or a heptadecyl group (that is, acyl group represented by R⁵-CO- is lauroyl group, myristoyl group, palmitoyl group or stearoyl group),
R⁶ is a hydrogen atom or a saturated or unsaturated, straight chain or branched chain hydrocarbon group having 7 - 10 carbon atoms,
m is 3 or 4,
n is an integer of 0 - 9,
R⁷ is a hydrogen atom or a saturated straight chain or branched chain hydrocarbon group having 1 - 4 carbon atoms, and R⁸ is a hydrogen atom.

### (compound-C)

A compound wherein R⁵ is an undecyl group (that is, acyl group represented by R⁵-CO- is lauroyl group),
R⁶ is a hydrogen atom or a saturated straight chain or branched chain hydrocarbon group having 7 - 10 carbon atoms,
m is 4,
n is an integer of 0 - 9,
R⁷ is a hydrogen atom, a methyl group or an ethyl group, and
R⁸ is a hydrogen atom.

Examples of the compound represented by the formula (2B) include the following.

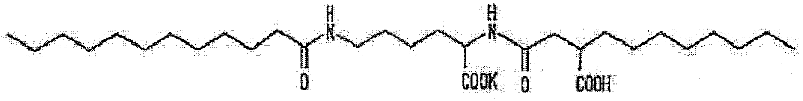

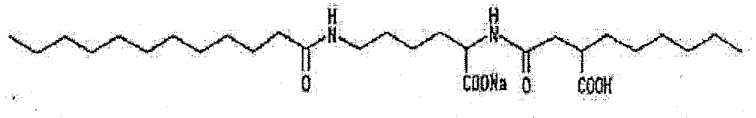

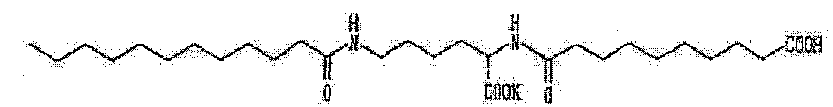

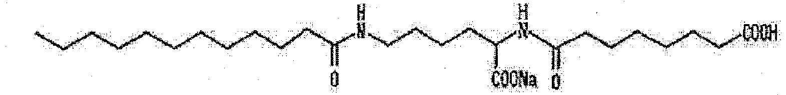

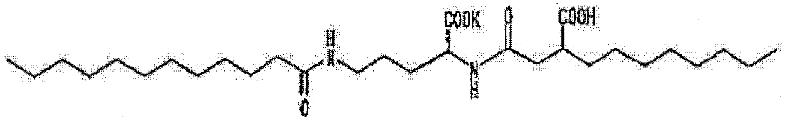

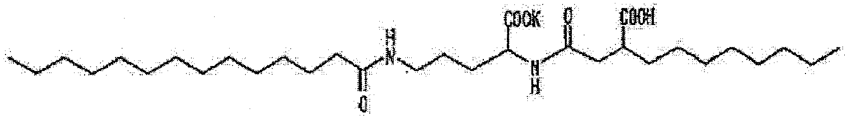

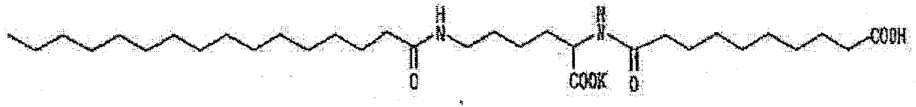

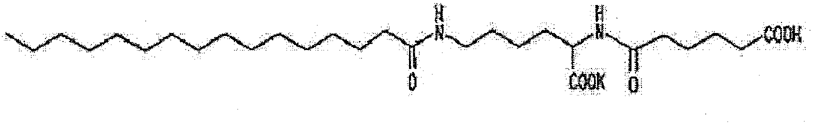

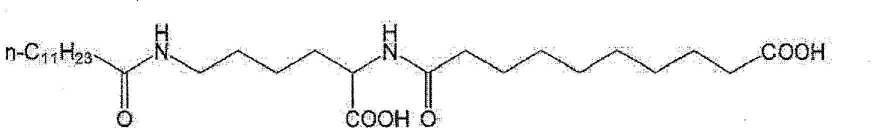

One or more kinds selected from the group consisting of Nε-lauroyl-Nα-(3-carboxytridecanoyl)-L-lysine ethyl ester, Nε-lauroyl-Nα-(3-carboxytridecanoyl)-L-lysine, Nε-lauroyl-Nα-(5-carboxypentanoyl)-L-lysine, Nε-lauroyl-Nα-(7-carboxyheptanoyl)-L-lysine, and Nε-lauroyl-Nα-(9-carboxynonanoyl)-L-lysine, or a salt thereof is/are preferable, and Nε-lauroyl-Nα-(9-carboxynonanoyl)-L-lysine or a salt thereof is more preferable.

A compound represented by the formula (2B) can be produced, for example, the method described in the aforementioned patent document 2 or a method analogous thereto. In addition, a compound represented by the formula (2B) can also be produced by hydrolyzing compound (5) in the production method of the aforementioned compound (1").

As a salt of a compound represented by the formula (2B), those similar to the salts exemplified as the salts of the compound represented by the formula (1) can be recited as examples.

In the gel composition of the present invention, the content of component (A) is preferably 0.03 - 5 wt%, more preferably 0.05 - 3 wt%, further preferably 0.1 - 2 wt%.

In the gel composition of the present invention, the content of component (B) is preferably 0.01 - 5 wt%, more preferably 0.02 - 3 wt%, further preferably 0.05 - 2 wt%.

In the gel composition of the present invention, the content of component (C) is preferably 50 - 99.9 wt%, more preferably 80 - 99.5 wt%, further preferably 85 - 98 wt%.

The present invention is useful since gelling is achieved by adding a small amount of a gelling agent (components (A) and (B)), as compared to conventional use of a water-soluble polymer as a gelling agent.

In the gel composition of the present invention, the weight ratio of component (A) and component (B) (component (A):component (B)) is preferably 99:1 - 30:70, more preferably 99:1 - 50:50, further preferably 95:5 - 50:50, further more preferably 75:25 - 50:50.

The gel composition of the present invention preferably has pH 4.5 - 7.5, more preferably 5 - 7.

The pH can be adjusted by a method known per se and, for example, the pH can be adjusted using citric acid, sodium hydroxide and the like.

The gel composition of the present invention is superior in the gelation ability and stability in the above-mentioned wide pH region. In the present invention, the gelation ability and stability can be evaluated, for example, according to the below-mentioned Experimental Example 1.

The gel composition of the present invention is a composition obtained by gelling an aqueous composition by using, as a gelling agent, a compound represented by the above-mentioned formula (1) or a salt thereof (component (A)) and a compound represented by the above-mentioned formula (2) or a salt thereof (component (B)) in combination.

In the present specification, a gelling agent refers to a substance that thickens a liquid or changes same to a jelly state or solid state. In the present specification, an aqueous composition means a composition containing water.

Examples of the aqueous composition include cosmetics, skin external preparations, quasi-drugs and the like, each containing water.

The gel composition of the present invention can be produced, for example, by the following method.

The gelling agent of the present invention is added to an aqueous composition, and the mixture is heated to 40 - 100°C as necessary, stirred to reach a uniform state, and the aqueous composition is gelated or thickened by standing at room temperature, whereby the gel composition of the present invention can be obtained. The hardness or viscosity of the gel can be freely adjusted by controlling the amount of the gelling agent of the present invention to be added.

The gel composition of the present invention can contain other gelling agent or solidifying agent as long as the effect of the present invention is not impaired. Examples of such other gelling agent or solidifying agent include natural polymers such as alginic acid, carrageenan, agar, guar gum, curdlan, xanthan gum, pullulan, gellan gum, gelatin, casein, albumin, collagen and the like, semisynthetic polymers such as methylcellulose, ethylcellulose, hydroxyethylcellulose, hydroxypropylcellulose, carboxymethylcellulose, methylhydroxypropylcellulose, soluble starch, carboxymethylstarch, methylstarch, propyleneglycol alginate and the like, synthetic polymers such as poly(vinyl alcohol), polyacrylate, polyvinylpyrrolidone, polyvinyl methyl ether, carboxyvinyl polymer, sodium polyacrylate, polyethyleneoxide, ethyleneoxide-propyleneoxide block copolymer and the like, inorganic substances such as bentonite, LAPONITE, fine powdered silicon oxide, colloidal alumina and the like, and the like.

The gel composition of the present invention can be utilized as gel cosmetics, gel skin external preparation, gel quasi-drug and the like by adding, besides the above-mentioned components, various components generally usable for cosmetics, skin external preparation or quasi-drug, to the extent that the effect of the present invention is not inhibited.

Examples of the component include oily component (oil agent), chelating agent, surfactant (e.g., nonionic surfactant), powder, amino acid, amino acid derivative, polyamino acid, lower alcohol (e.g., ethanol), higher alcohol, polyvalent alcohol, sugar alcohol and alkyleneoxide adduct thereof, water-soluble polymer, plant extract, nucleic acid, vitamin, enzyme, gelling agent, moisturizer (e.g., water-soluble moisturizing component), bactericidal agent and antibacterial agent, antiinflammatory agent, analgesic, antifungal agent, stratum corneum softening exfoliant, skin colorant, hormone agent, ultraviolet absorber, hair-growth agent, antiperspirant and astringent active ingredient, perspiration deodorant, vitamin, vasodilator, crude drug, pH adjuster, metal ion-sequestering agent, viscosity modifier, pearly sheen agent, natural flavor, synthetic flavor, dye, pigment, antioxidant, preservative, emulsifier, fat, wax, silicone compound, balm and the like.

Specific examples of the gel cosmetics in the present invention include adiaphoretic, facial cleanser, cleansing gel, milky lotion, massage cream, cold cream, moisture gel, facial mask, after shaving gel, foundation, chapstick, lip rouge, blush, mascara, shampoo, rinse, hair-growth agent, treatment, hair conditioner, tic, set lotion, hair cream, hair wax, hair mousse, permanent wave solution, hair dye, hair coloring, hair manicure, sunscreen oil, hand soap and aromatic and the like.

As the gel cosmetics in the present invention, gel cosmetics containing, besides components (A) - (C), (D) water-soluble moisturizing component (e.g., hyaluronate sodium, glycerol, sorbitol, PCA-Na (50% aqueous solution)), (E) water-soluble polymer (e.g., carboxyvinyl polymer, hydroxyethylcellulose, hydroxypropylethylcellulose, hydroxypropylstarch phosphate, xanthan gum), (F) oil agent (e.g., liquid paraffin, squalane, olive oil, shea butter, jojoba oil, isopropyl myristate, silicone oil, triethylhexanoin) and/or (G) nonionic surfactant (e.g., glyceryl laurate, coconut oil fatty acid sorbitan, polyoxyethylene hydrogenated castor oil, polyoxyethylene fatty acid glyceryl, cocamide DEA, sucrose laurate) can be mentioned.

The present invention also relates to a gelling agent containing (A) a compound represented by the above-mentioned formula (1) or a salt thereof, and (B) a compound represented by the above-mentioned formula (2B) or a salt thereof. Examples, weight ratio and the like of component (A) and component (B) are the same as those explained for the aforementioned gel composition.

### [Examples]

While the present invention is explained in more detail in the following by referring to Production Examples, Examples, Comparative Examples, Experimental Examples, the present invention is not limited thereto.

### [Production Example 1] Synthesis of bis(Nε-lauroyl-L-lysine)sebacoylamide

Nε-lauroyl-L-lysine (8.2 g, 25 mmol) was dissolved in a mixture of water (70 g) and 25% aqueous sodium hydroxide solution (10 g), and diethyl ether (80 g) was added. Sebacoyl chloride (3.3 g, 14 mmol) was slowly added to the ether layer. The two-layer solution was stirred for about for 1 hr while maintaining at 0°C, and then stirred at room temperature for 23 hr. A 75% sulfuric acid was added dropwise to adjust to pH 2, and the obtained white solid was collected by filtration and stirred with heating in acetonitrile solvent at 60°C for 1 hr. Hot filtration was performed while maintaining the mixture at 60°C, and the obtained white solid was dried to give bis(Nε-lauroyl-L-lysine)sebacoylamide (9.9 g).

### [Production Example 2] Synthesis of Nε-lauroyl-Nα-(9-carboxynonanoyl)-L-lysine

Nε-lauroyl-L-lysine (6.6 g, 20 mmol) was dissolved in a mixture of water (100 g) and 25% aqueous sodium hydroxide solution (16 g), and diethyl ether (50 g) was added. Methyl 10-chloro-10-oxodecanoate (3.5 g, 15 mmol) was slowly added to the ether layer. The two-layer solution was stirred for about for 1 hr while maintaining at 0°C, and then stirred at room temperature for 23 hr. Then, the temperature was raised to 60°C and, after stirring for 2 hr, the mixture was cooled to room temperature and adjusted to pH 2 by adding 75% sulfuric acid dropwise. The obtained white solid was collected by filtration, and recrystallized from acetonitrile to give Nε-lauroyl-Nα-(9-carboxynonanoyl)-L-lysine (5.7 g).

### [Examples 1 - 4 and Comparative Examples 1 - 2]

The compound obtained in Production Example 1 and the compound obtained in Production Example 2 were mixed and dispersed at room temperature at the ratios shown in Table 1 to achieve 1 mM concentration, and respectively adjusted to pH 5, pH 5.5, pH 6, pH 7 by using citric acid and sodium hydroxide to give the compositions of Examples 1 - 4 and Comparative Examples 1 - 2 (compositions adjusted to pH 5, pH 5.5, pH 6, pH 7 for each).

### [Experimental Example 1]

The compositions of Examples 1 - 4 and Comparative Examples 1 - 2 were tightly sealed completely in a micro test tube (2 mL, manufactured by Eppendorf) and a 20 mL glass bottle, stored at room temperature for 3 days or longer, and evaluated for the gelation ability and stability according to the following methods and criteria.

The results are shown in Table 1.

From the results of Table 1, it was shown that a composition containing the compound of Production Example 1 and the compound of Production Example 2 in combination is superior in the gelation ability and stability in a wide pH region.

### Evaluation 1: Gelation ability

A micro test tube containing a composition adjusted to each pH as mentioned above was placed upside down, visually observed, and evaluated by the following criteria.
⊙: does not fall even when glass bottle is upside down
○: thick and takes time to come down
×: liquid with no thickness like water

### Evaluation 2: Evaluation of appearance

A glass bottle containing a composition adjusted to each pH as mentioned above was visually observed and evaluated according to the following criteria.
⊙: transparent and uniform gel
○: slightly white turbid but uniform gel
×: gel is separated, or non-uniform precipitate is present
-: composition marked with × in Evaluation 1 is not evaluated

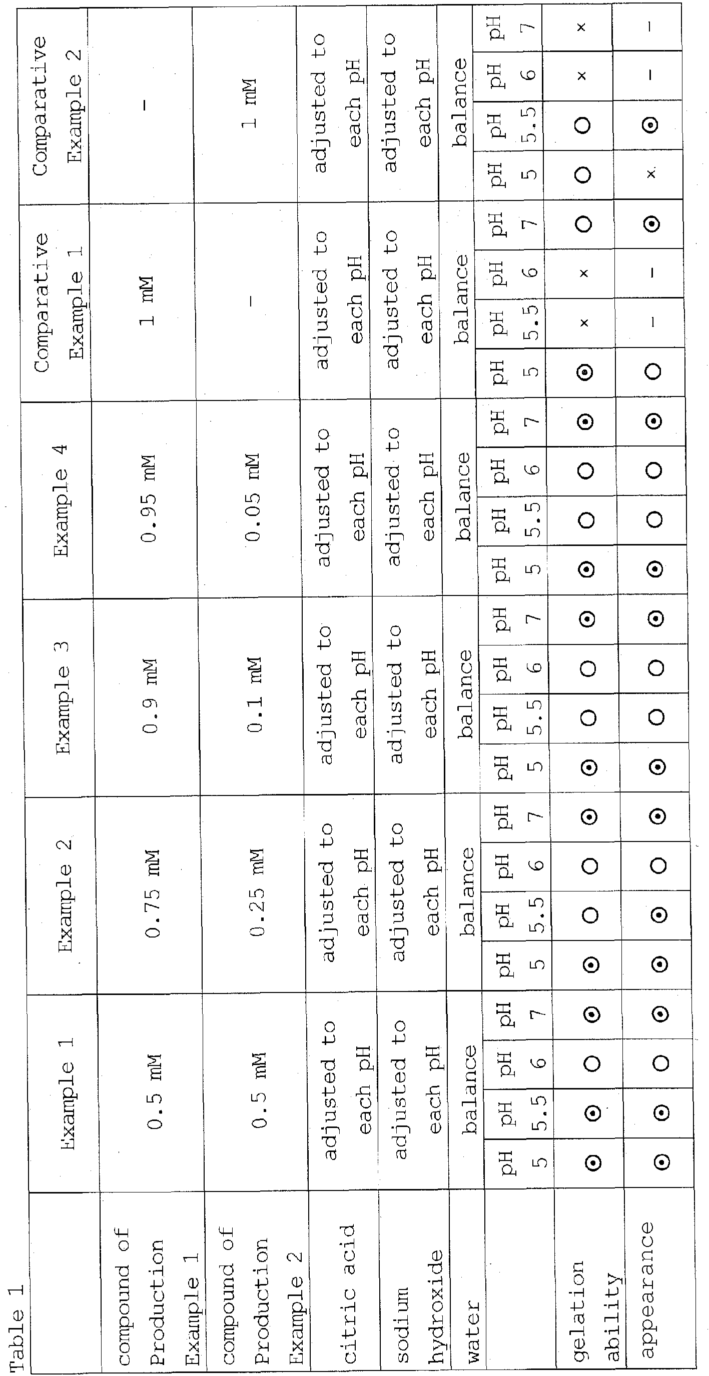

### [Example 5 and Comparative Example 3]

Respective components (wt%) were mixed by stirring at room temperature according to the compositions shown in Table 2. The mixtures were finally adjusted to pH 5.2 with citric acid to give the compositions of Example 5 and Comparative Example 3.

### [Experimental Example 2]

The compositions of Example 5 and Comparative Example 3 were tightly sealed completely in a micro test tube and 20 mL glass bottle, stored at room temperature for 3 days or longer, and the gelation ability and stability were evaluated by the same method and criteria as in Experimental Example 1.

The results are shown in Table 2.

From the results of Table 2, it was shown that a composition containing the compound of Production Example 1 and the compound of Production Example 2 in combination is superior in the gelation ability and stability .

**Table 2**

| | Example 5 | Comparative Example 3 |
|---|---|---|
| compound of Production Example 1 | 0.1 | 0.2 |
| compound of Production Example 2 | 0.1 | - |
| hyaluronic acid Na | 0.1 | 0.1 |
| glycerol | 5 | 5 |
| sorbitol | 5 | 5 |
| PCA-Na (50% aqueous solution) | 3 | 3 |
| ethanol | 5 | 5 |
| citric acid | pH adjusted | pH adjusted |
| water | to 100 | to 100 |
| gelation ability | ⊙ | ○ |
| appearance | ⊙ | × |

This application is based on patent application No. 2015-142305 filed in Japan, the contents of which are encompassed in full herein.

## Claims

1. A gel composition comprising
(A) a compound represented by the formula (1): 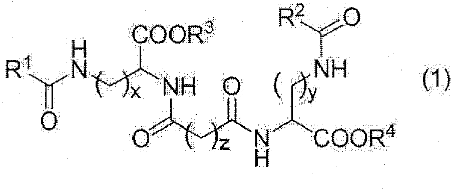 wherein
R¹ and R² are each independently an alkyl group having 5 - 21 carbon atoms or an alkenyl group having 5 - 21 carbon atoms,
R³ and R⁴ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof;
(B) a compound represented by the formula (2B): 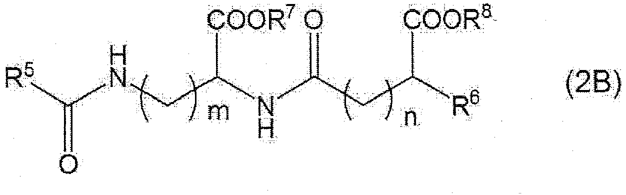 wherein
R⁵ is an alkyl group having 5 - 21 carbon atoms or an alkenyl group having 5 - 21 carbon atoms,
R⁶ is a hydrogen atom or an optionally substituted hydrocarbon group,
m is an integer of 1 - 4,
n is an integer of 0 - 15, and
R⁷ and R⁸ are each independently a hydrogen atom or an optionally substituted, saturated or unsaturated, straight chain or branched chain hydrocarbon group having 1 - 6 carbon atoms, or a salt thereof, and
(C) water.

2. The composition according to claim 1, wherein z is an integer of 0 - 10.

3. The composition according to claim 1 or 2, wherein z is an integer of 7 or 8.

4. The composition according to any one of claims 1 to 3, wherein x and y are each 4.

5. The composition according to any one of claims 1 to 4, wherein R¹ and R² are each independently a straight chain alkyl group having 5 - 15 carbon atoms.

6. The composition according to any one of claims 1 to 5, wherein R³ and R⁴ are each a hydrogen atom.

7. The composition according to any one of claims 1 to 6, wherein R⁵ is a straight chain alkyl group having 5 - 15 carbon atoms.

8. The composition according to any one of claims 1 to 7, wherein R⁶ is a hydrogen atom or an optionally substituted, saturated or unsaturated, straight chain or branched chain hydrocarbon group having 1 - 30 carbon atoms.

9. The composition according to any one of claims 1 to 8, wherein n is an integer of 0 - 9.

10. The composition according to any one of claims 1 to 9, wherein R⁷ and R⁸ are each independently a hydrogen atom or a saturated or unsaturated, straight chain or branched chain hydrocarbon group having 1 - 6 carbon atoms.

11. The composition according to any one of claims 1 to 10, wherein m is 4.

12. The composition according to any one of claims 1 to 11, wherein component (A) is bis(Nε-lauroyl-L-lysine)sebacoylamide, and
component (B) is Nε-lauroyl-Nα-(9-carboxynonanoyl)-L-lysine.

13. The composition according to any one of claims 1 to 12, having a pH of 5 - 7.

14. The composition according to any one of claims 1 to 13, wherein a weight ratio of (A) a compound represented by the formula (1) or a salt thereof and (B) a compound represented by the formula (2B) or a salt thereof ((A):(B)) is 99:1 - 50:50.

15. The composition according to any one of claims 1 to 14, which is a cosmetic.

16. A gelling agent comprising
(A) a compound represented by the formula (1): 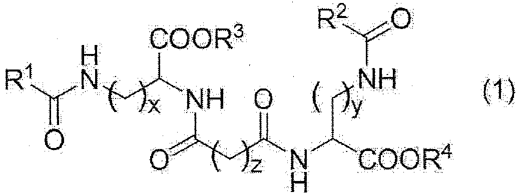 wherein
R¹ and R² are each independently an alkyl group having 5 - 21 carbon atoms or an alkenyl group having 5 - 21 carbon atoms,
R³ and R⁴ are each independently a hydrogen atom, an alkyl group having 1 - 22 carbon atoms or an alkenyl group having 2 - 22 carbon atoms,
z is an integer of not less than 0, and
x and y are each independently an integer of 2 - 4, or a salt thereof; and
(B) a compound represented by the formula (2B): 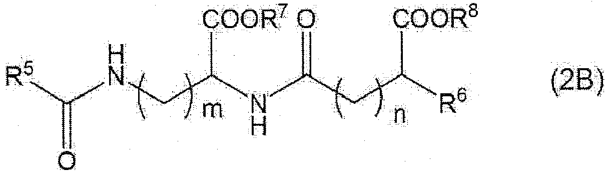 wherein
R⁵ is an alkyl group having 5 - 21 carbon atoms or an alkenyl group having 5 - 21 carbon atoms,
R⁶ is a hydrogen atom or an optionally substituted hydrocarbon group,
m is an integer of 1 - 4,
n is an integer of 0 - 15, and
R⁷ and R⁸ are each independently a hydrogen atom or an optionally substituted, saturated or unsaturated, straight chain or branched chain hydrocarbon group having 1 - 6 carbon atoms, or a salt thereof.

## Patentansprüche

1. Gelzusammensetzung, umfassend
(A) eine Verbindung der Formel (1): 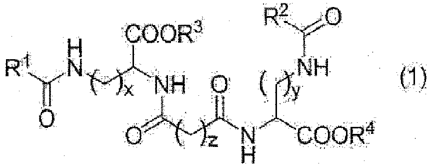 worin
R¹ und R² jeweils unabhängig voneinander eine Alkylgruppe mit 5 bis 21 Kohlenstoffatomen oder eine Alkenylgruppe mit 5 bis 21 Kohlenstoffatomen sind,
R³ und R⁴ jeweils unabhängig voneinander ein Wasserstoffatom, eine Alkylgruppe mit 1 bis 22 Kohlenstoffatomen oder eine Alkenylgruppe mit 2 bis 22 Kohlenstoffatomen sind,
z eine ganze Zahl von mindestens 0 ist und
x und y jeweils unabhängig voneinander eine ganze Zahl von 2 bis 4 sind, oder ein Salz davon;
(B) eine Verbindung der Formel (2B): 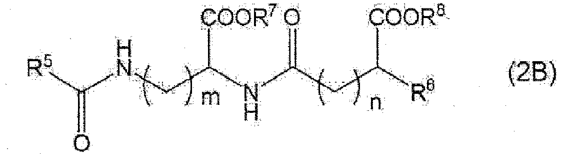 worin
R⁵ eine Alkylgruppe mit 5 bis 21 Kohlenstoffatomen oder eine Alkenylgruppe mit 5 bis 21 Kohlenstoffatomen ist,
R⁶ ein Wasserstoffatom oder eine gegebenenfalls substituierte Kohlenwasserstoffgruppe ist,
m eine ganze Zahl von 1 bis 4 ist,
n eine ganze Zahl von 0 bis 15 ist und
R⁷ und R⁸ jeweils unabhängig voneinander ein Wasserstoffatom oder eine gegebenenfalls substituierte, gesättigte oder ungesättigte, geradkettige oder verzweigtkettige Kohlenwasserstoffgruppe mit 1 bis 6 Kohlenstoffatomen sind, oder ein Salz davon und
(C) Wasser.

2. Zusammensetzung nach Anspruch 1, wobei z eine ganze Zahl von 0 bis 10 ist.

3. Zusammensetzung nach Anspruch 1 oder 2, wobei z die ganze Zahl 7 oder 8 ist.

4. Zusammensetzung nach einem der Ansprüche 1 bis 3, wobei x und y jeweils 4 sind.

5. Zusammensetzung nach einem der Ansprüche 1 bis 4, wobei R¹ und R² jeweils unabhängig voneinander eine geradkettige Alkylgruppe mit 5 bis 15 Kohlenstoffatomen sind.

6. Zusammensetzung nach einem der Ansprüche 1 bis 5, wobei R³ und R⁴ jeweils ein Wasserstoffatom sind.

7. Die Zusammensetzung nach einem der Ansprüche 1 bis 6,
worin R⁵ eine geradkettige Alkylgruppe mit 5 bis 15 Kohlenstoffatomen ist.

8. Zusammensetzung nach einem der Ansprüche 1 bis 7, worin R⁶ ein Wasserstoffatom oder eine gegebenenfalls substituierte, gesättigte oder ungesättigte, geradkettige oder verzweigtkettige Kohlenwasserstoffgruppe mit 1 bis 30 Kohlenstoffatomen ist.

9. Zusammensetzung nach einem der Ansprüche 1 bis 8, wobei n eine ganze Zahl von 0 bis 9 ist.

10. Zusammensetzung nach einem der Ansprüche 1 bis 9, wobei R⁷ und R⁸ jeweils unabhängig voneinander ein Wasserstoffatom oder eine gesättigte oder ungesättigte, geradkettige oder verzweigte Kohlenwasserstoffgruppe mit 1 - 6 Kohlenstoffatomen sind.

11. Zusammensetzung nach einem der Ansprüche 1 bis 10, wobei m gleich 4 ist.

12. Zusammensetzung nach einem der Ansprüche 1 bis 11, wobei die Komponente (A) Bis(Nε-lauroyl-L-lysin)sebacoylamid ist und die Komponente (B) ist Nε-Lauroyl-Nα-(9-carboxynonanoyl)-L-lysin ist.

13. Zusammensetzung nach einem der Ansprüche 1 bis 12 mit einem pH-Wert von 5 bis 7.

14. Zusammensetzung nach einem der Ansprüche 1 bis 13, wobei das Gewichtsverhältnis von (A) einer Verbindung der Formel (1) oder eines Salzes davon und (B) einer Verbindung der Formel (2B) oder eines Salzes davon ((A):(B)) 99:1 - 50:50 beträgt.

15. Zusammensetzung nach einem der Ansprüche 1 bis 14, die ein Kosmetikum ist.

16. Geliermittel, umfassend
(A) eine Verbindung der Formel (1) 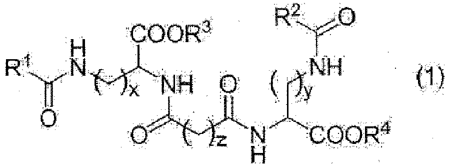 worin
R¹ und R² jeweils unabhängig voneinander eine Alkylgruppe mit 5 bis 21 Kohlenstoffatomen oder eine Alkenylgruppe mit 5 bis 21 Kohlenstoffatomen sind,
R³ und R⁴ jeweils unabhängig voneinander ein Wasserstoffatom, eine Alkylgruppe mit 1 bis 22 Kohlenstoffatomen oder eine Alkenylgruppe mit 2 bis 22 Kohlenstoffatomen sind,
z eine ganze Zahl von nicht weniger als 0 ist, und
x und y jeweils unabhängig voneinander eine ganze Zahl von 2 bis 4 sind, oder a Salz davon; und
(B) eine Verbindung der Formel (2B): 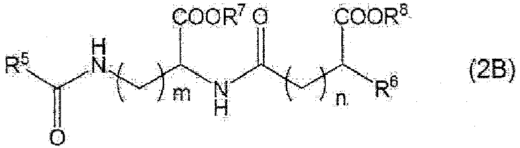 worin
R⁵ eine Alkylgruppe mit 5 bis 21 Kohlenstoffatomen oder ein Alkenylgruppe mit 5 bis 21 Kohlenstoffatomen ist,
R⁶ ein Wasserstoffatom oder eine gegebenenfalls substituierte Kohlenwasserstoffgruppe ist,
m eine ganze Zahl von 1 bis 4 ist,
n eine ganze Zahl von 0 bis 15 ist und
R⁷ und R⁸ jeweils unabhängig voneinander ein Wasserstoffatom oder eine gegebenenfalls substituierte, gesättigte oder ungesättigte, geradkettige oder verzweigtkettige Kohlenwasserstoffgruppe mit 1 bis 6 Kohlenstoffatomen sind, oder ein Salz davon.

## Revendications

1. Composition de gel comprenant
(A) un composé représenté par la formule (1) : 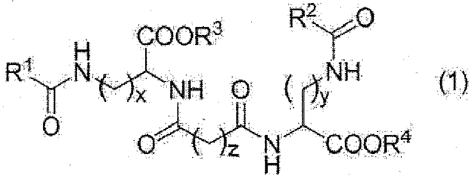 dans laquelle
R¹ et R² sont chacun indépendamment un groupe alkyle ayant de 5 à 21 atomes de carbone ou un groupe alcényle ayant de 5 à 21 atomes de carbone,
R³ et R⁴ sont chacun indépendamment un atome d'hydrogène, un groupe alkyle ayant de 1 à 22 atomes de carbone ou un groupe alcényle ayant de 2 à 22 atomes de carbone,
z est un entier supérieur ou égal à 0, et
x et y sont chacun indépendamment un entier de 2 à 4, ou un sel de celui-ci ;
(B) un composé représenté par la formule (2B) : 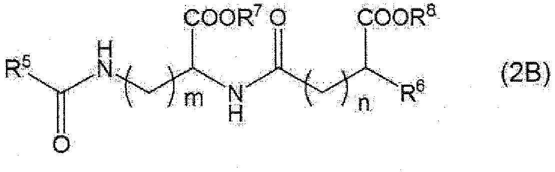 dans laquelle
R⁵ est un groupe alkyle ayant de 5 à 21 atomes de carbone ou un groupe alcényle ayant de 5 à 21 atomes de carbone,
R⁶ est un atome d'hydrogène ou un groupe hydrocarboné éventuellement substitué,
m est un entier de 1 à 4,
n est un entier de 0 à 15, et
R⁷ et R⁸ sont chacun indépendamment un atome d'hydrogène ou un groupe hydrocarboné à chaîne droite ou à chaîne ramifiée, saturé ou insaturé, éventuellement substitué ayant de 1 à 6 atomes de carbone, ou un sel de celui-ci, et
(C) de l'eau.

2. Composition selon la revendication 1, dans laquelle z est un entier de 0 à 10.

3. Composition selon la revendication 1 ou 2, dans laquelle z est un entier égal à 7 ou 8.

4. Composition selon l'une quelconque des revendications 1 à 3, dans laquelle x et y sont chacun 4.

5. Composition selon l'une quelconque des revendications 1 à 4, dans laquelle R¹ et R² sont chacun indépendamment un groupe alkyle à chaîne droite ayant 5 à 15 atomes de carbone.

6. Composition selon l'une quelconque des revendications 1 à 5, dans laquelle R³ et R⁴ sont chacun un atome d'hydrogène.

7. Composition selon l'une quelconque des revendications 1 à 6, dans laquelle R⁵ est un groupe alkyle à chaîne droite ayant 5 à 15 atomes de carbone.

8. Composition selon l'une quelconque des revendications 1 à 7, dans laquelle R⁶ est un atome d'hydrogène ou un groupe hydrocarboné à chaîne droite ou à chaîne ramifiée, saturé ou insaturé, éventuellement substitué ayant 1 à 30 atomes de carbone.

9. Composition selon l'une quelconque des revendications 1 à 8, dans laquelle n est un entier de 0 à 9.

10. Composition selon l'une quelconque des revendications 1 à 9, dans laquelle R⁷ et R⁸ sont chacun indépendamment un atome d'hydrogène ou groupe hydrocarboné à chaîne droite ou à chaîne ramifiée, saturé ou insaturé ayant 1 à 6 atomes de carbone.

11. Composition selon l'une quelconque des revendications 1 à 10, dans laquelle m est 4.

12. Composition selon l'une quelconque des revendications 1 à 11, dans laquelle le composant (A) est le bis(Nε-lauroyl-L-lysine)sébacoylamide, et
le composant (B) est la Nε-lauroyl-Nα-(9-carboxynonanoyl)-L-lysine.

13. Composition selon l'une quelconque des revendications 1 à 12, ayant un pH de 5 à 7.

14. Composition selon l'une quelconque des revendications 1 à 13, dans laquelle un rapport de poids de (A) un composé représenté par la formule (1) ou un sel de celui-ci et (B) un composé représenté par la formule (2B) ou un sel de celui-ci ((A):(B)) est 99:1 à 50:50.

15. Composition selon l'une quelconque des revendications 1 à 14, qui est un produit cosmétique.

16. Agent gélifiant comprenant
(A) un composé représenté par la formule (1) : 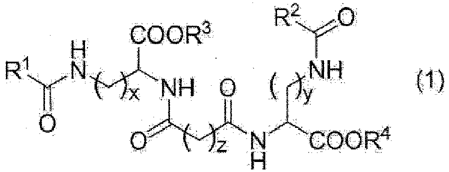 dans lequel
R¹ et R² sont chacun indépendamment un groupe alkyle ayant de 5 à 21 atomes de carbone ou un groupe alcényle ayant de 5 à 21 atomes de carbone,
R³ et R⁴ sont chacun indépendamment un atome d'hydrogène, un groupe alkyle ayant de 1 à 22 atomes de carbone ou un groupe alcényle ayant de 2 à 22 atomes de carbone,
z est un entier supérieur ou égal à 0, et
x et y sont chacun indépendamment un entier de 2 à 4, ou un sel de celui-ci ;
(B) un composé représenté par la formule (2B) : 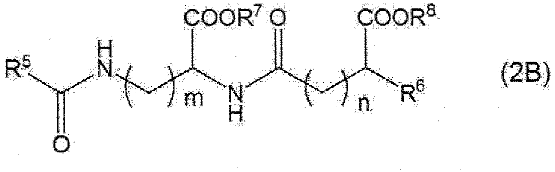 dans lequel
R⁵ est un groupe alkyle ayant de 5 à 21 atomes de carbone ou un groupe alcényle ayant de 5 à 21 atomes de carbone,
R⁶ est un atome d'hydrogène ou un groupe hydrocarboné éventuellement substitué,
m est un entier de 1 à 4,
n est un entier de 0 à 15, et
R⁷ et R⁸ sont chacun indépendamment un atome d'hydrogène ou un groupe hydrocarboné à chaîne droite ou à chaîne ramifiée, saturé ou insaturé, éventuellement substitué ayant de 1 à 6 atomes de carbone, ou un sel de celui-ci.
